# FASCICULE DE BREVET EUROPEEN

(11) **EP 1 149 813 B1**
(45) Date de publication et mention de la délivrance du brevet: **23.06.2004**
(21) Numéro de dépôt: 01400833.8
(22) Date de dépôt: 02.04.2001
(51) Int. Cl.: C07B 45/06, C07C 319/24, C07C 321/04, C07G 17/00

(54) **Procédé de fabrication d'olefines sulfurees**
Verfahren zur Herstellung von schwefelierten Olefinen
Process for the preparation of sulphurised olefins

(30) Priorité: 28.04.2000 FR 0005498
(43) Date de publication de la demande: 31.10.2001
(73) Titulaire: Atofina, 92091 Paris La Défense (FR)
(72) Inventeur: Luyendijk, Piet, 3233 AB Oostvoorne (NL); Devaux, Jean-Francois, 64110 Jurancon (FR); Monguillon, Bernard, 64270 Salies de Bearn (FR)
(74) Mandataire: Granet, Pierre

(56) Documents cités:
- EP-A- 0 342 454
- EP-A- 0 554 011

## Description

La présente invention concerne le domaine des oléfines sulfurées et a plus particulièrement pour objet un nouveau procédé de production d'oléfines sulfurées faiblement colorées par sulfuration au moyen de soufre et d'hydrogène sulfuré.

Les oléfines sulfurées sont des produits largement utilisés pour la sulfuration des catalyseurs et comme additifs pour lubrifiants ou pour élastomères. Ces produits sont composés essentiellement de mélanges de sulfures, disulfures et polysulfures organiques.

De nombreux procédés de production d'oléfines sulfurées et de polysulfures organiques sont connus de l'homme de l'art. Une première famille de procédés consiste à faire réagir un mercaptan et du soufre en présence d'un catalyseur basique. Ces procédés, décrits par exemple dans les brevets FR 2 607 496 et FR 2 630 104, sont coûteux car ils nécessitent l'utilisation de mercaptans qui doivent eux-mêmes être produits à partir d'oléfines ou d'alcools.

Le procédé décrit dans le brevet EP 342 454 pour produire des dialkyldisulfures et dialkylpolysulfures à partir d'oléfines est en fait un procédé en deux étapes où l'on effectue, dans un premier temps, une réaction H₂S + oléfine en présence d'un catalyseur solide pour former un mercaptan et où, dans un deuxième temps, ce mercaptan est mis en présence de soufre et d'un autre catalyseur hétérogène pour former un polysulfure. Ce procédé présente l'inconvénient de nécessiter deux étapes successives (2 réacteurs différents) avec des températures élevées.

D'autres procédés permettant d'obtenir des oléfines sulfurées ont été proposés :
**1)** La réaction oléfine + soufre en l'absence d'H₂S produit généralement des produits colorés. Pour éviter cet inconvénient, il a été proposé d'opérer en présence d'eau ou d'effectuer des lavages aqueux, mais ceci pose des problèmes de séparation de la phase aqueuse et d'élimination des effluents aqueux. De tels procédés sont décrits dans les brevets US 5 338 468, WO 92/03524, WO 92/00367, WO 97/24416, EP 714 970, EP 714 971 et FR 2 757 534. Dans tous ces procédés des températures élevées sont nécessaires pour mener à bien la réaction. Le brevet EP 201 197 décrit la réaction soufre + oléfine à une température de 140 à 180 °C.
**2)** La réaction oléfine + soufre + H₂S a été décrite dans les brevets EP 554 011, EP 889 030, US 4 119 549, US 4 119 550, US 4 191 659 et US 4 584 113 et la demande JP 11-246518. Cependant, les températures nécessaires pour mener à bien les réactions sont élevées (le plus souvent supérieures à 110°C pour qu'il y ait une conversion significative des réactifs) et/ou les pressions mentionnées sont le plus souvent très importantes.

Il a maintenant été trouvé un nouveau procédé de production d'oléfines sulfurées par sulfuration au moyen de soufre et d'H₂S en présence de catalyseurs solides, qui fournit des produits limpides et faiblement colorés et présente l'avantage de ne nécessiter que des températures peu élevées, généralement bien inférieures à 110°C, et de pouvoir être mis en oeuvre à des pressions peu élevées. De plus, l'utilisation de catalyseurs solides permet de séparer très facilement le catalyseur du produit fini ; une simple filtration permet la réutilisation des catalyseurs et l'obtention d'un produit fini stabilisé car exempt de catalyseurs. Ce procédé permet, de plus, de réaliser la réaction en une seule étape.

Le procédé selon l'invention pour la production d'oléfines sulfurées par sulfuration au moyen de soufre et d'hydrogène sulfuré est caractérisé en ce que la réaction est effectuée en présence d'un catalyseur solide acide et d'un catalyseur solide basique en une seule étape.

Une très large gamme de catalyseurs solides acides à utiliser selon l'invention peut convenir et comprend, par exemple, les différents polymères et copolymères à fonctions acides connus dans l'art comme résines échangeuses de cations, les zéolites à caractère acide, les silices-alumines, les zircones sulfatées ou les hétéropolyanions supportés, ou un mélange de ceux-ci.

A titre non limitatif, on peut citer les résines sulfonées à base de polystyrène réticulées (de préférence avec du divinylbenzène), les résines acryliques ou phénylacryliques à groupes carboxyliques libres, les résines du type phénol-formaldéhyde dérivées des acides phénol-sulfoniques, les échangeurs ligno-sulfoniques, etc..... Des résines de ce genre se trouvent dans le commerce sous différentes dénominations, en particulier Amberlite, Dowex, Diaion, Duolite, Levatit, ... Conviennent plus particulièrement les copolymères sulfonés du styrène avec le divinylbenzène. D'autre part, peuvent être employés avantageusement les copolymères de tétrafluoroéthylène avec un acide perfluorosulfonique connus sous la dénomination Nafion. De préférence, la résine solide acide sera utilisée aussi sèche que possible.

Une très large gamme de catalyseurs solides basiques à utiliser selon l'invention peut convenir et comprend, par exemple, les polymères ou copolymères à fonctions basiques bien connus dans l'art comme résines échangeuses d'anions, les zéolites à caractère basique, les alumines ou silices-alumines dopées par des métaux alcalins ou alcalino-terreux, ou un mélange de ces catalyseurs.

On peut citer à titre non limitatif les résines à base de polystyrène réticulé notamment avec du divinylbenzène, les résines acryliques ou phénylacryliques, les résines acryliques réticulées avec du divinylbenzène ou les résines du type phénolformaldéhyde. Ces résines portent des groupements fonctionnels amine primaire, secondaire ou tertiaire, ammonium quaternaire, polyéthylènepolyamine, guanidine ou amidine. De telles résines sont décrites par exemple dans les brevets FR 2 630 104, US 5 786 511, US 5 767 229, EP 931 789, US 5 726 253 et US 5 817 716.

Des résines échangeuses d'anions utilisables se trouvent dans le commerce sous différentes dénominations telles que, par exemple, Amberlite, Amberlyst, Diaion, Dowex, Duolite, Levatit, Reillex, ....

Dans le cas des résines à fonctions ammonium quaternaire, un préconditionnement avant usage peut s'avérer utile, tel un traitement à l'hydroxyde de sodium, suivi d'un lavage à l'eau et d'un séchage.

L'efficacité des résines échangeuses d'anions se trouve généralement améliorée lorsqu'on les utilise aussi sèches que possible.

Les zéolites à caractère basique utilisables selon l'invention sont des alumino-silicates caractérisés par une taille de pores bien définie et une grande surface spécifique. Ce peuvent être des zéolites de type X, Y ou L contenant au moins 3% massique d'un oxyde de métal alcalin (par exemple Na₂O) et de préférence plus de 10%. Le cation sodium peut aussi être remplacé par un autre cation alcalin.

Les oléfines à utiliser dans le procédé selon l'invention peuvent être choisies dans une très large gamme. Elles contiennent au moins une double liaison carbone-carbone qui n'est pas aromatique. Elles peuvent généralement être représentées par la formule : 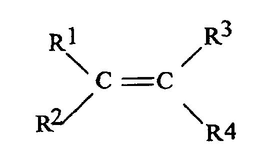 dans laquelle les symboles R¹, R², R³ et R⁴, identiques ou différents, représentent chacun un atome d'hydrogène, un radical aryle ou un radical alkyle, linéaire, ramifié ou cyclique, contenant de 1 à 20 atomes de carbones et pouvant comporter une ou plusieurs insaturations et/ou groupes aromatiques et/ou groupes OR⁵, SR⁵, NR⁵R⁶, CN, COR⁵, COOR⁵, CONR⁵R⁶, SiR⁵R⁶R⁷, Si(OR⁵)R⁶R⁷, Si(OR⁵)(OR⁶)R⁷, Si(OR⁵)(OR⁶)OR⁷, SO₂R⁵, SO₃R⁵, POR⁵R⁶, OP(O)(OR⁵)(OR⁶), NO₂ ou halogène, chacun des symboles R⁵, R⁶ et R⁷ désignant indépendamment un atome d'hydrogène ou un radical alkyle, cycloalkyle ou aryle comportant éventuellement une ou plusieurs insaturations.

Deux des symboles R¹, R², R³ et R⁴ peuvent aussi représenter un groupe alkylène éventuellement substitué, c'est-à-dire que la double liaison carbone-carbone peut être incluse dans un cycle comme, par exemple, dans le cyclohexène, le cyclopentadiène, le dicyclopentadiène... L'oléfine de l'invention peut également être un acide gras insaturé ou polyinsaturé, un ester d'acide gras insaturé ou polyinsaturé, un dérivé d'acide gras contenant au moins une double liaison, ou un mélange de ces derniers. Par exemple, ce peut être l'acide oléique, linoléique, linolénique, palmitoléique ou leurs esters d'origine naturelle comme les triglycérides ou d'origine synthétique comme, par exemple, les esters d'alcools aliphatiques ou de polyols. Ces acides ou ces esters gras peuvent être utilisés isolément ou en mélange comme dans les corps gras naturels, les huiles ou graisses végétales ou animales, ou leurs produits dérivés ; on peut citer par exemple l'huile de tournesol, de soja, de colza, de son de riz, de ricin, de suif, de tall oil, ... Dans le cadre de l'invention, les corps gras naturels ou leurs dérivés peuvent contenir une certaine proportion d'acides ou d'esters saturés qui se comportent comme des solvants dans les conditions de l'invention.

L'oléfine selon l'invention peut être également un terpène comme, par exemple, le pinène, le menthène, le limonène...

Des mélanges de plusieurs oléfines peuvent aussi être utilisés, comme par exemple un mélange de corps gras naturel avec une oléfine aliphatique non fonctionnalisée.

De préférence, on utilisera une oléfine telle que l'isobutylène, le diisobutylène, le triisobutylène, le tripropylène, le tétrapropylène, un acide gras, un ester gras, un mélange d'acides ou d'esters gras, ou une huile animale ou végétale éventuellement en mélange avec une oléfine aliphatique non fonctionnalisée.

De plus, les oléfines utilisées selon l'invention peuvent être diluées dans des solvants. En fin de réaction, ces solvants partent dans les évents ou sont séparés par distillation. De tels solvants peuvent être, par exemple, des hydrocarbures aliphatiques saturés comme le méthane, l'éthane, le propane, un butane ou un pentane. Le fait d'utiliser de tels mélanges d'oléfines et d'hydrocarbures saturés peut substantiellement améliorer l'économie du procédé selon l'invention dans la mesure où de telles charges peuvent être moins coûteuses qu'une charge d'oléfine relativement pure. Par exemple, on pourra utiliser une coupe contenant des hydrocarbures saturés et insaturés de 4 atomes de carbone à la place d'isobutylène pur.

Le soufre peut être utilisé sous forme solide, en pastille, en poudre ou sous forme liquide. Le ratio molaire soufre / oléfine peut aller de 0,4 : 1 à 2,5 : 1 et est de préférence compris entre 0,5 : 1 et 2 : 1.

Le ratio molaire H₂S / oléfine peut varier dans une large gamme (de 0,5 : 1 à 5 : 1, voire plus), mais de préférence, on utilise le minimum d'H₂S nécessaire pour que la réaction s'opère de façon satisfaisante, soit un ratio H₂S / oléfine compris entre 0,5 : 1 et 2 : 1.

Le procédé selon l'invention peut être mis en oeuvre en discontinu (batch) ou en continu.

L'efficacité catalytique des catalyseurs acides et basiques se manifeste généralement à partir d'une quantité minimale de 0,1 % en poids de chacun des deux catalyseurs par rapport à la quantité d'oléfine. Dans la plupart des cas, la quantité maximale utile est de l'ordre de 30 % en poids pour chacun des deux catalyseurs. Dans un procédé discontinu (batch), la quantité préférée est comprise entre 5 et 20 % pour chacun des deux catalyseurs.

Dans le cas d'une mise en oeuvre du procédé en continu, une charge de catalyseurs peut être utilisée pendant de longues périodes pour produire de grandes quantités de produits et le ratio massique de catalyseurs / oléfine n'a plus grande signification.

La proportion relative des deux catalyseurs peut varier dans une large mesure. Généralement, on utilise un ratio massique catalyseur acide / catalyseur basique compris entre 1 : 10 et 10 : 1 et, de préférence entre 1:2 et 2:1.

Le procédé selon l'invention peut être mis en oeuvre dans n'importe quel équipement adapté, par exemple, dans un réacteur, muni d'un agitateur, où les catalyseurs sont en suspension dans le milieu réactionnel liquide. Il peut aussi être mis en oeuvre au moyen d'un réacteur tubulaire dans lequel les catalyseurs sont disposés en lit fixe, en lit mobile ou en lit expansé ; dans ce cas, les catalyseurs acides et basiques peuvent être utilisés en mélange ou en couches alternées.

La réaction proprement dite peut avoir lieu dans un large domaine de température suivant les oléfines utilisées et le type de catalyseurs employés. Elle est en général effectuée à une température comprise entre 0 et 160°C, de préférence entre 0 et 110°C et, plus particulièrement entre 20 et 90°C.

La réaction est conduite à une pression adaptée à la conversion de l'oléfine. On opère avantageusement entre 1 et 50 bars absolus, de préférence entre 1 et 20 bars absolus. La pression peut varier au cours de la réaction notamment en fonction du profil de température et de l'avancement de la réaction.

Avantageusement, après une phase sous pression en réacteur fermé où l'oléfine est convertie, le ciel du réacteur est mis à pression atmosphérique ou en dépression de façon à éliminer l'H₂S excédentaire et à finir de convertir le mercaptan formé. On peut envisager dans cette dernière phase d'introduire un gaz inerte (par exemple du méthane, de l'air ou de l'azote) de façon à entraîner les composés volatiles résiduels tel l'H₂S ou l'oléfine résiduelle.

Dans le cas d'une réaction par batch, en fin de réaction, le mélange de catalyseurs peut être récupéré par simple filtration et réutilisé.

Si l'on désire diminuer son odeur, le stabiliser ou réduire sa corrosivité, le produit soufré peut être traité par toute méthode connue par l'homme de l'art. De telles méthodes sont décrites par exemple dans les brevets JP 58140063, US 5 155 275, US 5 206 439, US 5 218 147, US 5 403 961, US 5 457 234, US 5 530163, US 5 559 271, US 5 091 112, US 5 174 922, US 5 208 382, US 5 242 613, EP 76376, et EP 933 358.

Dans les exemples suivants qui illustrent l'invention sans la limiter, la mesure de l'oléfine résiduelle dans un mélange réactionnel a été effectuée par chromatographie gaz, et la mesure du mercaptan a été effectuée par argentimétrie. Dans les exemples 1 à 7, les résultats de la mesure du mercaptan ont été ramenés à une masse molaire de 202 (correspondant à celle du mercaptan dérivé du tétrapropylène).

### EXEMPLE 1

Dans un réacteur en acier inoxydable de 1 litre inerté à l'azote, on a introduit 20 g de résine acide Amberlyst 15 (Rôhm & Haas), qui est une résine polystyrène-divinylbenzène macroréticulée à fonctions -SO₃H présentant une surface spécifique d'environ 50 m²/g et 1,8 mmoles de fonctions sulfonique par ml de résine, et 25 g de la résine basique DETA (polystyrène-divinylbenzène à fonctions diéthylènetriamine) dont la préparation est décrite à l'exemple 1 du brevet EP 931 789.

Après addition de 64 g de soufre (2,0 moles) et 168 g de tétrapropylène (1,0 mole), on a ajouté à 5°C 27 g d'hydrogène sulfuré (0,8 mole) puis on a porté la température à 90°C sous agitation vigoureuse.

On a atteint une pression maximale de 7 bars absolus. Après 3 heures, le réacteur a été relié à la torche et, après avoir ramené la pression à 1,1 bar absolu, on a effectué un stripping à l'azote pendant 3 heures.

Après une simple filtration sur une grille métallique située en fond de réacteur, on a obtenu une huile visqueuse limpide de couleur jaune ; les résines restent à l'intérieur du réacteur.

Les analyses (voir tableau I) indiquent que la majorité du tétrapropylène (noté TP) a réagi et que le produit contient très peu de mercaptan.

### EXEMPLE 2

Sur la charge de résines qui a servi à l'exemple 1, on a additionné à nouveau du soufre, du tétrapropylène et de l'hydrogène sulfuré et effectué une seconde opération dans les mêmes conditions que précédemment. On a obtenu exactement les mêmes résultats qu'à l'exemple 1.

### EXEMPLE 3

On a reproduit l'exemple 1, mais en utilisant un ratio molaire initial TP/S/H₂S de 1/1,9/1,2.

Les résultats indiqués dans le tableau I montrent que l'utilisation d'une quantité d'H₂S plus importante n'apporte qu'une très légère amélioration dans la conversion du tétrapropylène et induit une augmentation de la pression du réacteur.

### EXEMPLE 4 (comparatif)

On a reproduit l'exemple 3, mais sans employer de catalyseur.

Les résultats sont reportés dans le tableau I. Ils montrent que, malgré la pression élevée atteinte, le tétrapropylène ne réagit pas de façon significative à la température de 90°C. On retrouve des quantités importantes de soufre solide dans le mélange réactionnel.

### EXEMPLE 5 (comparatif)

On a reproduit l'exemple 3, mais en remplaçant le mélange de catalyseurs hétérogènes (A15 + DETA) par le catalyseur homogène n-butylamine.

Ni à 90°C, ni à 120°C on n'a détecté de disparition du tétrapropylène. Il faut chauffer à plus de 130°C (avec une pression de l'ordre de 21-22 bars) pour commencer à détecter une conversion significative du tétrapropylène après 3 heures.

### EXEMPLE 6 (comparatif)

On a reproduit l'exemple 3, mais en utilisant la résine basique DETA comme seul catalyseur. On constate (voir tableau 1) que le tétrapropylène n'a pas réagi pas de façon significative. On retrouve des quantités importantes de soufre solide dans le mélange réactionnel.

### EXEMPLE 7 (comparatif)

On a reproduit l'exemple 3, mais en utilisant la résine acide Amberlyst A15 comme seul catalyseur.

Le produit obtenu contient une très forte proportion de tétrapropylène résiduel et de mercaptan. On retrouve des quantités importantes de soufre solide dans le mélange réactionnel.

**TABLEAU 1 :**

| **Réaction du tétrapropylène avec le soufre et l'H2S** | | | | | | |
|---|---|---|---|---|---|---|
| Exemple | Catalyseur(s) | Ratio TP/S/H₂S (moles) | P.maxi (bars absolus) | Température (°C) | TP Résiduel % masse | Mercaptan % masse |
| 1 | A15 + DETA | 1/2/0,8 | 7 | 90 °C | 10 % | 0,9% |
| 2 | A15 + DETA | 1/2/0,8 | 7 | 90 °C | 10 % | 0,9% |
| 3 | A15 + DETA | 1/1,9/1,2 | 13 | 90 °C | 8 % | 0,9% |
| 4* | Sans | 1/1,9/1,2 | 18 | 90 °C | > 99 % | < 0,1 % |
| 5* | n C₄H₉NH₂ | 1/1,9/1,2 | 20 | 120 °C | > 99 % | < 0,1 % |
| 6* | DETA | 1/1,9/1,2 | 18 | 90 °C | > 99 % | < 0,1 % |
| 7* | A15 | 1/1,9/1,2 | 7 | 90 °C | 52 % | 15% |

| | | | | | | |
|---|---|---|---|---|---|---|
| * Exemples comparatifs | | | | | | |

### EXEMPLE 8

Dans le même réacteur qu'à l'exemple 1, on a introduit 30 g de résine Amberlyst A15 et 30 g de résine DETA, puis 100 g de soufre (3,1 mol) et 177 g d'isoprène (2,6 mol). On a ajouté 72 g d'H₂S (2,1 mol) en 25 minutes à 20-25°C, puis augmenté progressivement la température à 40°C (3 h) et ensuite à 60°C(4 h). La pression maximale atteinte a été de 12 bars absolus.

L'analyse d'un prélèvement montre que le mélange réactionnel contenait alors 0,7 % d'isoprène et 4,0 % en masse de mercaptan (exprimé par rapport à une masse moléculaire de 108). ,

Après 2 heures de stripping à l'azote à 60°C à pression atmosphérique, on ne détectait plus d'isoprène et le taux de mercaptan était de 1,1 %. Après filtration sur une grille métallique en fond de réacteur, on a obtenu 294 g d'une huile jaune limpide dont la teneur en soufre est de 48 % en masse.

### EXEMPLE 9

Dans le même réacteur qu'à l'exemple 1, on a introduit 30 g de résine Amberlyst A15 et 30 g de résine basique Amberlyst A21 (vendue par Röhm & Haas), qui est une résine polystyrène-divinylbenzène de type macroporeux à fonctions -CH₂N(CH₃)₂ présentant une surface spécifique d'environ 25 m²/g et 1,3 mmoles de fonctions amine tertiaire par ml de résine. On a introduit ensuite 154 g de soufre (4,8 mol) et 336 g de diisobutylène (3,0 mol). On a alors ajouté 84 g d'H₂S (2,5 mol) en 60 minutes à 30°C. On a porté la température à 60°C et laissé réagir pendant 3 heures. La pression maximale atteinte a été de 9 bars absolus.

L'analyse d'un prélèvement montre que le mélange réactionnel contenait à ce stade 2 % de diisobutylène et 3,4 % en masse de mercaptan (exprimé par rapport à une masse moléculaire de 146).

Après 2 heures de stripping à l'azote à 60°C à pression atmosphérique, la teneur en diisobutylène était de 2 % et celle de mercaptan de 0,7 %. Après filtration sur une grille métallique en fond de réacteur, on a obtenu 558 g d'une huile jaune limpide dont la teneur en soufre est de 36,7 % en masse.

### EXEMPLE 10

Dans le même réacteur qu'à l'exemple 1, on a introduit 30 g de résine Amberlyst A15 et 30 g de zéolite LZY-52 sous forme d'extrudés (vendue par UOP), qui est une zéolite de type Y échangée contenant typiquement 10,4 % d'oxyde de sodium, 0,3% d'oxyde de calcium, 0,2 % d'oxyde de fer III, 66,5 % de silice et 20,8 % d'alumine et présentant une surface spécifique d'environ 820 m²/g. On a introduit ensuite 153 g de soufre (4,8 mol) et 336 g de diisobutylène (3,0 mol). On a alors ajouté 82 g d'H₂S (2,4 mol) en 50 minutes à 20-30°C. On a porté la température à 88°C et laissé réagir pendant 4 heures. La pression maximale atteinte a été de 12 bars absolus.

L'analyse d'un prélèvement montre que le mélange réactionnel contenait à ce stade 3,0 % de diisobutylène et 3,9 % en masse de mercaptan (exprimé par rapport à une masse moléculaire de 146).

Après 2 heures de stripping à l'azote à 60°C à pression atmosphérique, la teneur en diisobutylène était de 4 % et celle de mercaptan de 1,5 %. Après filtration sur une grille métallique en fond de réacteur, on a obtenu une huile jaune limpide.

### EXEMPLE 11

Dans le même réacteur qu'à l'exemple 1, on a introduit 18 g de résine Amberlyst A15 et 18 g de résine DETA, puis 60 g de soufre (1,9 mol) et 122 g d'isobutylène (2,2 mol). On a ajouté 69 g d'H₂S (2,0 mol) en 70 minutes à 30°C, puis laissé réagir à cette température pendant 3 heures. La pression maximale atteinte a été de 5,5 bars absolus.

L'analyse d'un prélèvement liquide montre que le mélange réactionnel contenait à ce stade 0,2 % d'isobutylène et 32 % de t-butylmercaptan (exprimés en pourcentage d'aire sur le chromatogramme).

Le contenu du réacteur a ensuite été détendu. L'analyse des gaz issus de cette détente montrait une teneur en isobutylène de 0,6 % massique. La phase liquide dans le réacteur contenait 0,15 % massique d'isobutylène. Au total, la conversion de l'isobutylène est d'environ 99 %.

Après 2 heures de stripping à l'azote à 60°C, le produit avait une teneur en isobutylène de 0,1 % et en mercaptan (ramené à une masse molaire de 92) de 1,1 %.

Après filtration sur une grille métallique en fond de réacteur, le produit brut avait l'aspect d'une huile jaune limpide.

### EXEMPLE 12

Dans le même réacteur qu'à l'exemple 1, on a introduit 30 g de résine Amberlyst A15 et 30 g de résine DETA, puis 77 g de soufre (2,4 mol) et 196 g de d'oxyde de mésityle (2,0 mol). On a ajouté 54 g d'H₂S (1,6 mol) en 30 minutes à 30°C, puis laissé réagir 2 heures à cette température puis 2 heures à 60°C. La pression maximale atteinte a été de 5,5 bars absolus.

L'analyse d'un prélèvement liquide montre que le mélange réactionnel ne contenait plus d'oxyde de mésityle en quantité détectable. La teneur en mercaptan (ramené à une masse molaire de 130) était de 6.3%.

Le contenu du réacteur a été détendu. Après 4 heures de stripping à l'azote à 70°C, le produit avait une teneur de 0,6 % massique de mercaptan (ramené à une masse molaire de 130).

Après filtration sur une grille métallique en fond de réacteur, le produit brut avait l'aspect d'une huile jaune limpide.

### EXEMPLE 13

Dans le même réacteur qu'à l'exemple 1, on a introduit 30 g de résine Amberlyst A15 et 30 g de résine DETA, puis 46 g de soufre (1,4 mol), 202 g de n-dodécène (1,2 mol). On a ajouté 33 g d'H₂S (1,0 mol) en 25 minutes à 25°C. On a laissé réagir à 65°C pendant 3 heures puis à 80°C température pendant 3 heures. La pression maximale atteinte a été de 8 bars absolus.

Le contenu du réacteur a été détendu. Après 3 heures de stripping à l'azote à 80°C, le produit avait une teneur de 1,5 % massique de mercaptan (ramené à une masse molaire de 202) et de 8,5 % massique de n-dodécène.

Après filtration sur une grille métallique en fond de réacteur, le produit brut avait l'aspect d'une huile jaune limpide.

### EXEMPLE 14

Dans le même réacteur qu'à l'exemple 1, on a introduit 30 g de résine Amberlyst A15 et 30 g de résine DETA, puis 38 g de soufre (1,2 mol), 268 g d'alcool oléique (1,0 mol). On a ajouté 27 g d'H₂S (0,8 mol) en 15 minutes à 25°C. On a laissé réagir à 70°C pendant 4 heures. La pression maximale atteinte a été de 9 bars absolus.

Le contenu du réacteur a été détendu. Après 4 heures de stripping à l'azote à 60°C, le produit avait une teneur de 1,8 % massique de mercaptan (ramené à une masse molaire de 202) et de 5,1 % massique d'alcool oléique.

Après filtration sur une grille métallique en fond de réacteur, le produit brut avait l'aspect d'une huile jaune limpide.

### EXEMPLE 15

Dans le même réacteur qu'à l'exemple 1, on a introduit 30 g de résine Amberlyst A15 et 30 g de zéolite LZY-54 sous forme d'extrudés (vendue par UOP), qui est une zéolite de type Y échangée contenant typiquement 10 % d'oxyde de sodium, 0,23 % d'oxyde de fer III, 66 % de silice et 21 % d'alumine et présentant une surface spécifique d'environ 750 m²/g. On a introduit ensuite 28,4 g de soufre (0,89 mol) et 250 g d'oléate de méthyle technique vendu par FINA CHEMICALS et contenant 55 % massique d'oléate de méthyle (soit 0,46 mol). On a alors porté la température à 126°C et ajouté en continu de l'hydrogène sulfuré pour maintenir une pression de 13 bars absolus. Après 9 heures de réaction dans ces conditions, la consommation d'hydrogène sulfuré était de 33 g.

Après 1 heure de stripping à l'azote à pression atmosphérique, la teneur en oléate de méthyle était de 4,6 %. Après filtration, on a obtenu 255 g d'une huile jaune-orangé. Aucun dépôt de soufre solide n'a été observé ni dans la phase liquide, ni sur le filtre.

### EXEMPLE 16

Dans le même réacteur qu'à l'exemple 1, on a introduit 30 g de résine Amberlyst A15 et 30 g de zéolite LZY-54. On a introduit ensuite 28,4 g de soufre (0,89 mol) et 250 g d'un mélange d'esters méthyliques, vendu par ATOFINA sous le nom d'ESTEROL C et provenant du cracking du ricinoléate de méthyle, ce mélange contenant 29 % de linoléate de méthyle (0,24 mol), 50 % d'oléate de méthyle (0,42 mol), ainsi que du stéarate et du palmitate de méthyle. On a alors porté la température à 126°C et ajouté en continu de l'hydrogène sulfuré pour maintenir une pression de 9 bars absolus. Après 12 heures de réaction dans ces conditions, la consommation d'hydrogène sulfuré était de 27 g.

Après 2 heures de stripping à l'azote à pression atmosphérique à 80°C, la teneur en oléate de méthyle était de 3,2 % et on ne détectait plus de linoléate de méthyle. Après filtration, on a obtenu 261 g d'une huile jaune-orangé. Aucun dépôt de soufre solide n'a été observé ni dans la phase liquide, ni sur le filtre.

## Revendications

1. Procédé de fabrication d'oléfines sulfurées à partir d'oléfine(s), de soufre et d'hydrogène sulfuré, **caractérisé en ce que** la réaction est effectuée en présence d'un catalyseur solide acide choisi parmi une résine échangeuse de cations, une zéolite à caractère acide, une silice-alumine, une zircone sulfatée, un hétéropolyanion supporté, ou un mélange de ces solides et en présence d'un catalyseur solide basique choisi parmi une résine échangeuse d'anions, une zéolite à caractère basique, une alumine ou silice-alumine dopée par des métaux alcalins ou alcalino-terreux, ou un mélange de ces catalyseurs.

2. Procédé selon la revendication 1 dans lequel le catalyseur solide acide est une résine échangeuse de cations, de préférence une résine sulfonée à base d'un polystyrène réticulé avec du divinylbenzène.

3. Procédé selon l'une des revendications 1 à 2, dans lequel le catalyseur solide basique est une résine échangeuse d'anions, de préférence une résine à base de polystyrène réticulée avec du divinylbenzène et porteuse de groupements fonctionnels amine, ammonium quaternaire, polyéthylènepolyamine, guanidine ou amidine.

4. Procédé selon l'une des revendications 1 à 3 dans lequel le rapport molaire soufre / oléfine(s) va de 0,4 : 1 à 2,5 : 1 et est, de préférence, compris entre 0,5: 1 et 2 : 1.

5. Procédé selon l'une des revendications 1 à 4 dans lequel le rapport molaire H₂S / oléfine(s) va de 0,5 : 1 à 5 : 1 et est, de préférence, compris entre 0,5:1 et2:1.

6. Procédé selon l'une des revendications 1 à 5 dans lequel chacun des deux catalyseurs est utilisé en une quantité allant de 0,1 à 30 % en poids par rapport au poids d'oléfine(s) et, de préférence, comprise entre 5 et 20 %.

7. Procédé selon l'une des revendications 1 à 6 dans lequel le rapport massique catalyseur acide/catalyseur basique est compris entre 1:10 et 10:1 et, de préférence entre 1:2 et 2:1.

8. Procédé selon l'une des revendications 1 à 7 dans lequel on opère à une température allant de 0 à 160°C, de préférence comprise entre 0 et 110°C et, plus particulièrement, entre 20 et 90°C.

9. Procédé selon l'une des revendications 1 à 8 dans lequel on opère à une pression allant de 1 à 50 bars absolus, de préférence comprise entre 1 et 20 bars absolus.

10. Procédé selon l'une des revendications 1 à 9 dans lequel la ou les oléfines sont choisies parmi celles de formule : 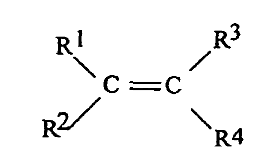 dans laquelle les symboles R¹, R², R³ et R⁴, identiques ou différents, représentent chacun un atome d'hydrogène, un radical aryle ou un radical alkyle, linéaire, ramifié ou cyclique, contenant de 1 à 20 atomes de carbone et pouvant comporter une ou plusieurs insaturations et/ou groupes aromatiques et/ou groupes OR⁵, SR⁵, NR⁵R⁶, CN, COR⁵, COOR⁵, CONR⁵R⁶, SiR⁵R⁶R⁷, Si(OR⁵)R⁶R⁷, Si(OR⁵)(OR⁶)R⁷, Si(OR⁵)(OR⁶)OR⁷, SO₂R⁵, SO₃R⁵, POR⁵R⁶, OP(O)(OR⁵)(OR⁶), NO₂ ou halogène, chacun des symboles R⁵, R⁶ et R⁷ désignant indépendamment un atome d'hydrogène ou un radical alkyle, cycloalkyle ou aryle comportant éventuellement une ou plusieurs insaturations, deux des symboles R¹, R², R³ et R⁴ pouvant aussi représenter un groupe alkylène éventuellement substitué.

11. Procédé selon la revendication 10 dans lequel on utilise l'isobutylène, le diisobutylène, le triisobutylène, le tripropylène ou le tétrapropylène.

12. Procédé selon la revendication 10 dans lequel on utilise un acide gras, un ester gras, un mélange d'acides ou d'esters gras, ou une huile animale ou végétale.

13. Procédé selon la revendication 10 dans lequel on utilise un mélange d'oléfine aliphatique non fonctionnalisée avec un acide gras, un ester gras ou une huile animale ou végétale.

## Patentansprüche

1. Verfahren zur Herstellung von geschwefelten Olefinen aus Olefin(en), Schwefel und Schwefelwasserstoff, **dadurch gekennzeichnet, daß** man die Umsetzung in Gegenwart eines festen sauren Katalysators, der unter einem Kationenaustauscherharz, einem Zeolith mit saurem Charakter, einem Siliciumoxid-Aluminiumoxid, einem sulfatierten Zirconiumoxid, einem geträgerten Heteropolyanion oder einem Gemisch dieser Feststoffe ausgewählt ist, und in Gegenwart eines festen basischen Katalysators, der unter einem Anionenaustauscherharz, einem Zeolith mit basischem Charakter, einem Aluminiumoxid oder Siliciumoxid-Aluminiumoxid mit Alkalimetall- oder Erdalkalimetalldotierung oder einem Gemisch dieser Katalysatoren ausgewählt ist, duchführt.

2. Verfahren nach Anspruch 1, bei dem man als festen sauren Katalysator ein Kationenaustauscherharz, vorzugsweise ein sulfoniertes Harz auf Basis von mit Divinylbenzol vernetztem Polystyrol, verwendet.

3. Verfahren nach einem der Ansprüche 1 bis 2, bei dem man als festen basischen Katalysator ein Anionenaustauscherharz, vorzugsweise ein Harz auf Basis von mit Divinylbenzol vernetztem Polystyrol mit Amin-, quaternären Ammonium-, Polyethylenpolyamin-, Guanidin- oder Amidinfunktionen, verwendet.

4. Verfahren nach einem der Ansprüche 1 bis 3, bei dem das Schwefel/Olefin-Molverhältnis 0,4:1 bis 2,5:1 beträgt und vorzugsweise zwischen 0,5:1 und 2:1 liegt.

5. Verfahren nach einem der Ansprüche 1 bis 4, bei dem das H₂S/Olefin-Molverhältnis 0,5:1 bis 5:1 beträgt und vorzugsweise zwischen 0,5:1 und 2:1 liegt.

6. Verfahren nach einem der Ansprüche 1 bis 5, bei dem man jeden der beiden Katalysatoren in einer Menge von 0,1 bis 30 Gew.-%, bezogen auf das Gewicht des Olefins bzw. der Olefine, und vorzugsweise zwischen 5 und 20% verwendet.

7. Verfahren nach einem der Ansprüche 1 bis 6, bei dem das Gewichtsverhältnis von saurem Katalysator zu basischem Katalysator zwischen 1:10 und 10:1 und vorzugsweise zwischen 1:2 und 2:1 liegt.

8. Verfahren nach einem der Ansprüche 1 bis 7, bei dem man bei einer Temperatur von 0 bis 160°C, vorzugsweise zwischen 0 und 110°C und insbesondere zwischen 20 und 90°C arbeitet.

9. Verfahren nach einem der Ansprüche 1 bis 8, bei dem man bei einem Druck von 1 bis 50 bar absolut und vorzugsweise zwischen 1 und 20 bar absolut arbeitet.

10. Verfahren nach einem der Ansprüche 1 bis 9, bei dem man das Olefin bzw. die Olefine unter denjenigen der Formel: 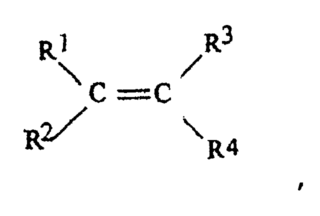 worin die Symbole R¹, R², R³ und R⁴ gleich oder verschieden sind und jeweils für ein Wasserstoffatom, einen Arylrest oder einen linearen, verzweigten oder cyclischen Alkylrest, der 1 bis 20 Kohlenstoffatome enthält und eine oder mehrere Ungesättigtheiten und/oder aromatische Gruppen und/oder OR⁵-, SR⁵-, NR⁵R⁶-, CN-, COR⁵-, COOR⁵-, CONR⁵R⁶-, SiR⁵R⁶R⁷-, Si(OR⁵)R⁶R⁷-, Si(OR⁵)(OR⁶)R⁷-, Si(OR⁵)(OR⁶)OR⁷-, SO₂R⁵-, SO₃R⁵-, POR⁵R⁶-, OP(O)(OR⁵)(OR⁶)-, NO₂- oder Halogengruppen enthalten kann, wobei jedes der Symbole R⁵, R⁶ und R⁷ unabhängig für ein Wasserstoffatom oder einen Alkyl-, Cycloalkyl- oder Arylrest, der gegebenenfalls eine oder mehrere Ungesättigtheiten enthalten kann, steht und zwei der Symbole R¹, R², R³ und R⁴ auch für eine gegebenenfalls substituierte Alkylengruppe stehen können, auswählt.

11. Verfahren nach Anspruch 10, bei dem man Isobutylen, Diisobutylen, Triisobutylen, Tripropylen oder Tetrapropylen verwendet.

12. Verfahren nach Anspruch 10, bei dem man eine Fettsäure, einen Fettsäureester, ein Gemisch von Fettsäuren oder Fettsäureestern oder ein tierisches oder pflanzliches Öl verwendet.

13. Verfahren nach Anspruch 10, bei dem man ein Gemisch aus nicht funktionalisiertem aliphatischem Olefin und einer Fettsäure, einem Fettsäureester oder einem tierischen oder pflanzlichen Öl verwendet.

## Claims

1. Process for manufacturing sulphurized olefins from olefin(s), sulphur and hydrogen sulphide, **characterized in that** the reaction is carried out in the presence of a solid acid catalyst chosen from a cation-exchange resin, a zeolite of acid character, a silica-alumina, a sulphated zirconia, a supported heteropolyanion, or a mixture of these solids, and in the presence of a solid basic catalyst chosen from an anion-exchange resin, a zeolite of basic character, an alumina or silica-alumina doped with alkali metals or with alkaline earth metals, or a mixture of these catalysts.

2. Process according to Claim 1, in which the solid acid catalyst is a cation-exchange resin, preferably a sulphonated resin based on a polystyrene crosslinked with divinylbenzene.

3. Process according to either of Claims 1 and 2, in which the solid basic catalyst is an anion-exchange resin, preferably a resin based on polystyrene crosslinked with divinylbenzene and bearing amine, quaternary ammonium, polyethylene polyamine, guanidine or amidine functional groups.

4. Process according to one of Claims 1 to 3, in which the molar ratio sulphur/olefin(s) is from 0.4:1 to 2.5:1 and is preferably between 0.5:1 and 2:1.

5. Process according to one of Claims 1 to 4, in which the molar ratio H₂S/olefin(s) is from 0.5:1 to 5:1, preferably between 0.5:1 and 2:1.

6. Process according to one of Claims 1 to 5, in which each of the two catalysts is utilized in an amount of from 0.1 to 30% by weight with respect to the weight of olefin(s), and preferably between 5 and 20%.

7. Process according to one of Claims 1 to 6, in which the ratio by weight acid catalyst/basic catalyst is between 1:10 and 10:1, and preferably between 1:2 and 2:1.

8. Process according to one of Claims 1 to 7, in which operations are carried out at a temperature of from 0 to 160°C, preferably between 0 and 110°C and more particularly between 20 and 90°C.

9. Process according to one of Claims 1 to 8, in which operations are carried out at a pressure of from 1 to 50 bar absolute, preferably between 1 and 20 bar absolute.

10. Process according to one of Claims 1 to 9, in which the olefin(s) are selected from those of the formula: 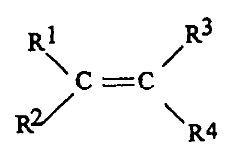 in which each of symbols for R¹, R², R³ and R⁴, which are identical or different, represents a hydrogen atom, an aryl radical or an alkyl radical, linear, branched or cyclic, which contains from 1 to 20 carbon atoms and may contain one or more unsaturations and/or aromatic groups and/or OR⁵, SR⁵, NR⁵R⁶, CN, COR⁵, COOR⁵, CONR⁵R⁶, SiR⁵R⁶R⁷, Si(OR⁵)R⁶R⁷, Si(OR⁵)(OR⁶)R⁷, Si(OR⁵)(OR⁶)OR⁷, SO₂R⁵, SO₃R⁵, POR⁵R⁶, OP(O)(OR⁵)(OR⁶), NO₂ or halogen groups, and each of the symbols for R⁵, R⁶ and R⁷ denotes independently an atom of hydrogen or an alkyl, cycloalkyl or aryl radical optionally containing one or more unsaturations, and two of the symbols for R¹, R², R³ and R⁴ may also represent an unsubstituted or substituted alkylene group.

11. Process according to Claim 10, in which use is made of isobutylene, diisobutylene, triisobutylene, tripropylene or tetrapropylene.

12. Process according to Claim 10, in which use is made of a fatty acid, a fatty ester, a mixture of fatty esters or acids, or an animal or vegetable oil.

13. Process according to Claim 10, in which use is made of a mixture of non-functionalized aliphatic olefin with a fatty acid, a fatty ester or an animal or vegetable oil.
